# EUROPEAN PATENT APPLICATION

(11) **EP 4 538 310 A1**
(43) Date of publication of application: **16.04.2025**
(21) Application number: 22921104.0
(22) Date of filing: 20.01.2022
(51) Int. Cl.: C08G 63/664, C08G 63/78, A61K 9/00, A61K 9/06, A61K 31/428, A61K 31/439, A61K 45/00, A61K 47/34, A61P 1/08, A61P 25/16

(54) **SERIES OF POLYMERS HAVING PROPERTIES OF ACID-SENSITIVE DEGRADATION AND TEMPERATURE SENSITIVITY AND DRUG LOADING COMPOSITION THEREOF**

(71) Applicant: NANJING FANTAI CHEMICAL PHARMACEUTICAL RESEARCH INSTITUTE, Nanjing, Jiangsu 210037 (CN)
(72) Inventor: HE, Dongsheng, Nanjing, Jiangsu 210037 (CN); TU, Jiasheng, Nanjing, Jiangsu 210037 (CN); FENG, Zuoxun, Nanjing, Jiangsu 210037 (CN); SUN, Chunmeng, Nanjing, Jiangsu 210037 (CN)
(74) Representative: Lermer, Christoph
(86) International application number: PCT/CN2022/072942
(87) International publication number: WO 2023/137659

(57) **Abstract**

Disclosed are a series of polymers having the properties of acid-sensitive degradation and temperature sensitivity and a drug loading composition thereof. The polymers have a structure represented by formula (1) and have the properties of degradation and temperature sensitivity in a physiological environment, that is, the polymers are in a solid state at a temperature below a phase change temperature, and are in a liquid state at a temperature above the phase change temperature, so that the polymers are in a stable state in a low-temperature environment of a storage condition and are in a state of relatively high flowability in a use environment, and thus ensure storage stability and needle penetration during injection while maintaining good drug release properties and degradability. Also disclosed are a preparation method of the polymers and a pharmaceutical composition containing the polymers.

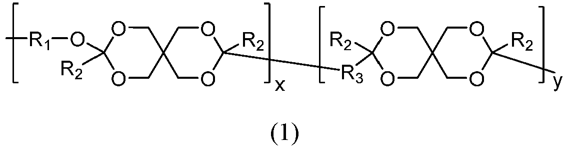

## Description

### TECHNICAL FIELD

The present invention belongs to the technical field of biomedicine and polymer medical materials, and specifically relates to a series of polymers having the properties of acid-sensitive degradation and positive temperature sensitivity and a preparation method and pharmaceutical composition thereof.

### BACKGROUND ART

In the field of drug sustained and controlled release research, the properties of sustained-release materials are one of the important factors determining the success or failure of formulation design. The ideal sustained release injection material should simultaneously consider good biocompatibility, effective control of drug release, and good injectability. In the field of drug delivery, commonly used in situ gel include natural gel materials and synthetic gel materials according to their sources. These materials form gel in situ after injection through reverse temperature sensitivity (within a certain temperature range, they are sols at low temperature, and perform cross-linking to generate gel when the temperature rises), high temperature positive temperature sensitivity (they are sols at high temperature, and generate gel when the temperature drops after injection), pH sensitivity, chemical cross-linking, or solvent exchange mechanisms, which act as a drug reservoir and slowly release drugs, and the drug release mechanisms include diffusion and corrosion. However, the above traditional in situ gel materials have more or less the following problems: poor biocompatibility and difficult metabolism; chemical crosslinking monomers have certain toxic effects; unstable drug release properties; high water content in the system increases the risk of deterioration; traditional positive temperature-sensitive materials injected at excessively high temperatures can cause pain to patients and even lead to local necrosis.

Poly(ortho ester) is a class of high molecular weight substances rich in ortho ester bonds. The ortho ester bonds in its structure can be hydrolyzed in aqueous environments, making it has excellent biodegradability. Due to its excellent biodegradability and surface corrosion properties, poly(ortho ester) has achieved rapid development in the field of drug sustained and controlled release, and is currently being used in the marketed formulation Sustol^{®} developed by Heron Therapeutics.

A classic poly(ortho ester) can be presented in a semi-solid form, and related products are marketed in the form of pre-filled injections. However, its rheological properties are still insufficient. The classic semi-solid poly(ortho ester) has high viscosity and low flowability at ambient temperatures, which results in poor needle penetration during injection administration and increases the difficulty of injection administration when used alone as an in situ sustained release matrix. Therefore, when using the classic semi-solid poly(ortho ester) as a sustained release carrier for injection administration, it is necessary to mix it with a certain proportion of viscosity modifiers (such as dimethyl sulfoxide, N-methylpyrrolidone, and other organic solvents) to improve needle penetration. However, the introduction of these viscosity modifiers increases the risk of adverse reactions such as irritation and toxicity. In addition, the changes in the rheological properties of the classic semi-solid poly(ortho ester) are greatly limited by temperature, and it still has a certain degree of flowability under low temperature conditions during storage. Therefore, the pre-filled injections may experience aggregation of drug particles, and crystallization and precipitation of drugs during long-term storage, which can have adverse effects on the quality of the formulation.

Currently, patents related to the use of poly(ortho ester) polymers as drug carriers mainly include: ① combination of poly(ortho ester) and proton inert solvent (application number: CN201480028192); ② long-term polymer delivery system (application number: CN201580033564); and ③ novel poly(ortho ester) pharmaceutical excipient and novel formulation of sustained release drug thereof (application number: CN201210436124). Patents ① and ② are patents applied by Heron Therapeutics in the United States regarding an application of poly(ortho ester) as a drug sustained-release carrier. The poly(ortho ester) used is a traditional semi-solid poly(ortho ester). Although this semi-solid poly(ortho ester) has certain effects in drug release control, its rheological properties are shortcomings that limit its use. Simply using classical semi-solid poly(ortho ester) has high viscosity and low flowability in the usage environment, which is difficult to meet the injection needs. The above-mentioned patent provides a pharmaceutical composition containing a "viscosity regulator" to solve the problem of difficult injection of pharmaceutical compositions. However, suitable viscosity regulators (including organic solvents such as dimethyl sulfoxide, N-methylpyrrolidone, dimethylacetamide, etc.) may increase the irritation and toxicity of the pharmaceutical composition while improving its flowability, thereby increasing the risk of formulation application. However, a traditional poly(ortho ester) still exhibits certain flowability at a storage temperature, which poses risks of crystallization and precipitation of a drug from the composition. In addition, relevant US patents (such as US10398686, US10357570, US10213510, etc.) have similar content to the above-mentioned patents ① and ②.

Patent ③ provides a novel poly(ortho ester) with a structure different from a traditional poly(ortho ester) and a related sustained release formulation. This patent focuses on a synthesis method of a novel polymer rich in a poly(ortho ester) structure. In addition to a traditional poly(ortho ester) synthesis pathway, it provides a novel poly(ortho ester) structure that also has biodegradability and a synthesis pathway thereof, and also looks forward to an application of this polymer as a pharmaceutical excipient, especially as a drug sustained-release carrier. However, the unique degradation pathway of this novel poly(ortho ester) poses higher safety risks than the traditional poly(ortho ester). During the degradation process of this novel poly(ortho ester), one of the final products of the degradation of a five-membered ring of ortho ester is formic acid, and this degradation product with visual neurotoxicity greatly limits its clinical application prospects.

Compared with the above-mentioned inventions, the novel polymer containing an ortho ester structure provided by the present invention creatively introduces the properties of positive temperature sensitivity while maintaining high safety and excellent drug release properties of a traditional poly(ortho ester), and allows a pharmaceutical composition prepared from the polymer to maintain excellent drug release properties and degradability, while also taking into account the stability of the composition during storage and needle penetration during injection. In summary, this type of polymer and a drug delivery system thereof have the advantages of stable storage, convenient administration, stable drug release, and controllable degradation, and have significant value for application and research in the pharmaceutical field.

### SUMMARY OF THE INVENTION

One objective of the present invention is to provide a series of polymers having the properties of acid-sensitive degradation and positive temperature sensitivity and a preparation method thereof. This series of polymers has advantages such as acid-sensitive degradation, positive temperature sensitivity, and good biocompatibility. This preparation method has a simple process and is easy to control.

Another objective of the present invention is to provide an oligomer monomer and a preparation method thereof. This oligomer monomer can be used to synthesize the above-mentioned series of polymers having the properties of acid-sensitive degradation and positive temperature sensitivity, and has the characteristics of simple synthesis, wide raw material sources, and low cost.

Another objective of the present invention is to provide a drug complex comprising the above-mentioned series of polymers having the properties of acid-sensitive degradation and positive temperature sensitivity as a sustained release matrix.

Therefore, the present invention provides a polymer having properties of temperature sensitivity and a structure represented by formula (1), 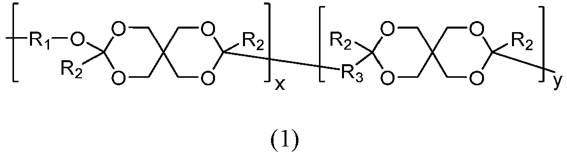
wherein: x and y are independently integers greater than 1;
R₁ is: 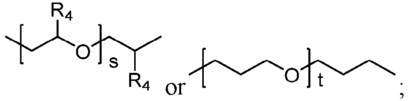
wherein:
   s is an integer from 0 to 30;
   t is an integer from 0 to 30;
   R₄ is hydrogen or methyl;
   R₂ is C₁₋₄ alkyl;
   R₃ is: 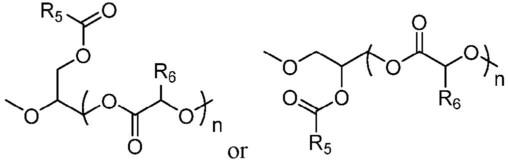
   wherein:
      R₅ is C₁₋₃₀ alkyl with or without a functional group, wherein the functional group is independently selected from a carbon-carbon double bond, a carbon-carbon triple bond, carbonyl, an aldehyde group, carboxyl, an ester bond, an amide bond, an ether bond, and amino;
      R₆ is hydrogen or C₁₋₄ alkyl;
      n is an integer from 1 to 20.

The polymer according to the present invention is preferably polymerized from the following three monomers:
monomer A: 3,9-di(ethylidene)-2,4,8,10-tetraoxaspiro[5,5]undecane or a homologue thereof;
monomer B: a diol; and
monomer C: an oligomer monomer of formula (2) or (3): 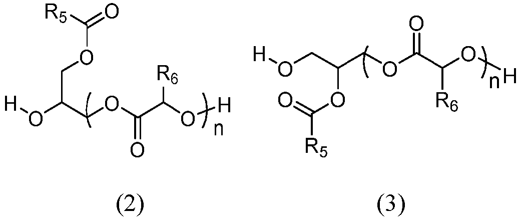
wherein:
   R₅ is C₁₋₃₀ alkyl with or without a functional group, wherein the functional group is independently selected from a carbon-carbon double bond, a carbon-carbon triple bond, carbonyl, an aldehyde group, carboxyl, an ester bond, an amide bond, an ether bond, and amino;
   R₆ is hydrogen or C₁₋₄ alkyl;
   n is an integer from 1 to 20.
   The polymer according to the present invention, more preferably, wherein
   the monomer A: 3,9-di(ethylidene)-2,4,8,10-tetraoxaspiro[5,5]undecane; 3,9-di(propylidene)-2,4,8,10-tetraoxaspiro[5.5]undecane;
   monomer B: a diol (triethylene glycol or tripropylene glycol); and
   in the monomer C:
   R₅ is C₁₋₃₀ alkyl with or without a functional group, wherein the functional group is independently selected from a carbon-carbon double bond, a carbon-carbon triple bond, carbonyl, an aldehyde group, carboxyl, and an ester bond;
   R₆ is hydrogen or C₁₋₂ alkyl;
   n is an integer from 1 to 15.

The polymer according to the present invention, particularly preferably, wherein
the monomer A: 3,9-di(ethylidene)-2,4,8,10-tetraoxaspiro[5,5]undecane;
in the monomer C:
R₅ is C₁₋₃₀ alkyl with or without a functional group, wherein the functional group is independently selected from a carbon-carbon double bond, a carbon-carbon triple bond, and carbonyl;
R₆ is hydrogen or C₁₋₂ alkyl;
n is an integer from 1 to 10.

The most preferred polymer according to the present invention is the following polymer: 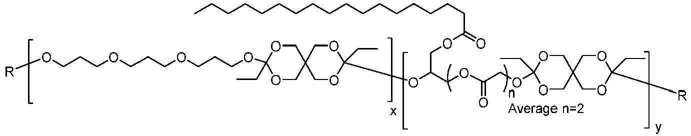 wherein: x and y are independently integers greater than 1.

The present invention further provides a preparation method of the polymer according to the present invention, which includes polymerization is performed on monomer A: 3,9-di(ethylidene)-2,4,8,10-tetraoxaspiro[5,5]undecane or a homologue thereof; monomer B: a diol (triethylene glycol or tripropylene glycol); and monomer C: an oligomer monomer of formula (2) or (3): 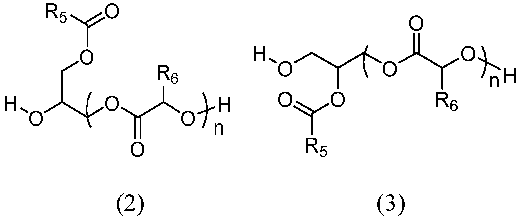 wherein:
R₅ is C₁₋₃₀ alkyl with or without a functional group, wherein the functional group is independently selected from a carbon-carbon double bond, a carbon-carbon triple bond, carbonyl, an aldehyde group, carboxyl, an ester bond, an amide bond, an ether bond, and amino;
R₆ is hydrogen or C₁₋₄ alkyl; and
n is an integer from 1 to 20;
in the presence of an organic solvent at a reaction temperature of 0°C-300°C.

The polymer according to the present invention has a molecular weight of 1000-50000, preferably 1000-20000.

The polymer according to the present invention has a composite viscosity of not less than 10000 Pa·s at a storage temperature and not more than 500 Pa·s at a use temperature.

The polymer according to the present invention has the properties of temperature sensitivity, and the temperature at which its storage modulus value and loss modulus value are equal is between 4°C and 40°C.

The preparation method of the polymer according to the present invention, wherein a molar ratio of the monomer B and the monomer C (based on the monomer A as 100%) is independently 0.01%-99.99%.

The preparation method according to the present invention occurs in the presence of a reaction solvent, preferably a polar non-proton solvent, more preferably ethyl acetate, tetrahydrofuran, acetonitrile, dimethyl sulfoxide, or a mixture of multiple solvents thereof. The reaction temperature is 0°C-300°C, preferably 20°C-80°C.

The present invention further comprises a composition of the polymer according to the present invention.

The composition contains one or more therapeutic active agents. The therapeutic active agent is a substance used for preventing, treating, and diagnosing human diseases, purposefully regulating human physiological functions, and specifying indications or major functions, usage, and dosage, and comprises traditional Chinese medicines, chemical drugs, and biological products;
the therapeutic active agent is an antiparkinsonian drug, and selected from levodopa, carbidopa, nitecapone, bromocriptine, pramipexole, ropionitril, selegiline, benzoxetine, phenytoin, amantadine, and rotigotine;
the therapeutic active agent is an antiemetic agent, and selected from diphenhydramine, metoclopramide, scopolamine, benzoxetine, chlorpromazine, ondansetron, granisetron, metoclopramide, and domperidone;
the therapeutic active agent is a local anesthetic, and selected from procaine, tetracaine, lidocaine, and bupivacaine;
the therapeutic active agent is a nonsteroidal anti-inflammatory drug selected from aspirin, acetaminophen, indomethacin, ibuprofen, naproxen, and meloxicam;
the therapeutic active agent is a growth factor;
the therapeutic active agent is a gene-based drug; and
the therapeutic active agent is a protein drug or therapeutic peptide, and selected from insulin and glucagon-like peptide.

A preparation method of the pharmaceutical composition according to the present invention includes the steps of mixing, encapsulating, chelating or compounding the polymer and the therapeutic active agent, wherein a preparation temperature is 0°C-300°C, preferably, the preparation temperature is 20°C-120°C.

On the basis of obtaining the polymer according to the present invention, the present invention also discloses an oligomer monomer of structural formula (2) or (3): 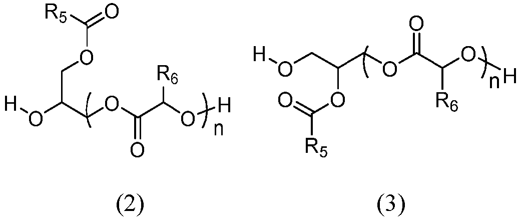 wherein:
R₅ is C₁₋₃₀ alkyl with or without a functional group, wherein the functional group is independently selected from a carbon-carbon double bond, a carbon-carbon triple bond, carbonyl, an aldehyde group, carboxyl, an ester bond, an amide bond, an ether bond, and amino;
R₆ is hydrogen or C₁₋₄ alkyl;
n is an integer from 1 to 20.

A preparation method of the oligomer monomer, wherein the oligomer is prepared by polymerization of monomer D having the following structural formula (6) or (7) and monomer E (ethylene or propylene) under heating conditions; 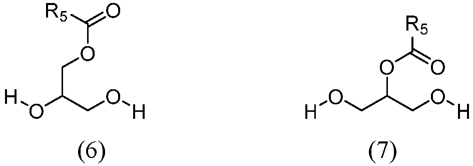 wherein:
R₅ is C₁₋₃₀ alkyl with or without a functional group, wherein the functional group is independently selected from a carbon-carbon double bond, a carbon-carbon triple bond, carbonyl, an aldehyde group, carboxyl, an ester bond, an amide bond, an ether bond, and amino.
A molar ratio of components D and E is 2:1 to 1:5;
preferably, a heating temperature is 80°C-260°C. A reaction time is 0.5 h-120 h.

The specific preparation method of the polymer according to the present invention includes the following steps:
adding DETOSU, a diol, and oligomer monomer C as shown in formula (2) or (3) in an appropriate proportion to the reaction; under strict anhydrous and anaerobic conditions, dissolving the DETOSU in a suitable reaction solvent and the diol, and dissolving the oligomer monomer C as shown in formula (2) or (3) in the reaction solvent; adding a solution of the oligomer monomer C as shown in formula (2) or (3) to a solution of the DETOSU and the diol to initiate the reaction; within a few minutes, the reaction solution reaches its boiling point; and cooling the solution to room temperature and then removing the solvent by rotary evaporation at 50°C-80°C.

The specific preparation method of the oligomer monomer according to the present invention includes the following steps:
a molar ratio of glycerol monoester to glycolide (or lactide) is 2:1 to 1:5; and under inert gas protection, adding the glycerol monoester and the glycolide (or lactide) into a reaction vessel, and stirring in a closed mode at 80°C-260°C for 6 h-72 h without catalyst or solvent.

The specific pharmaceutical composition according to the present invention includes:
(i) the polymer according to claim 1; and
(ii) one or more therapeutic active agents dispersed or dissolved in the polymer according to claim 1;
the active agent is released from the composition within a specified time.

### The following further illustrates the beneficial effects of the present invention through experimental data

**Table 1. Changes in the rheological properties of the novel polymer provided by the present invention in the range of 0°C-40°C**

| Temperature °C | Storage modulus Pa | Loss modulus Pa | Complex viscosity Pa·s |
|---|---|---|---|
| 0.0 | 218664 | 82318.4 | 74371.8 |
| 2.5 | 385619 | 102964 | 127046 |
| 5.0 | 289686 | 89595.7 | 96519.3 |
| 7.5 | 265772 | 107411 | 91245.4 |
| 10.0 | 270637 | 126571 | 95102.1 |
| 12.5 | 278405 | 106905 | 94927.9 |
| 15.0 | 318311 | 115521 | 107788 |
| 17.5 | 290672 | 96074.9 | 97446.8 |
| 20.0 | 288675 | 82273.2 | 95547.2 |
| 22.5 | 222696 | 61755.3 | 73561.3 |
| 25.0 | 129053 | 33610.8 | 42449.2 |
| 27.5 | 35692.5 | 10750.8 | 11865.5 |
| 30.0 | 4375.99 | 1609.25 | 1484.12 |
| 32.5 | 1651.35 | 617.153 | 561.15 |
| .35.0 | 810.559 | 323.273 | 277.772 |
| 37.5 | 186.394 | 72.4604 | 63.6566 |
| 40.0 | 2.69006 | 13.7245 | 4.45176 |

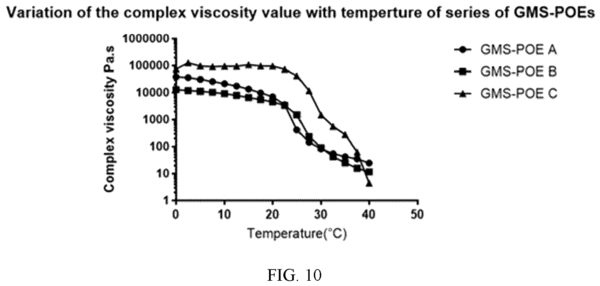

After research, we were surprised to discover a class of novel polymers containing an ortho ester structure, as shown in Table 1 (the polymer used in Example 2 was used in the above-mentioned experiment), which had the properties of positive temperature sensitivity. By adjusting the proportion of block units inside polymer molecules, the storage modulus and loss modulus of the polymers could be flexibly adjusted to make their values equal at a specific temperature within the range of 20°C-40°C. At the same time, as the ambient temperature gradually rose above this specific temperature, as shown in FIG. 10, the composite viscosity of the polymers also sharply decreased, greatly improving the flowability of the polymers. The rheological properties of these polymers, which were sensitive to temperature changes, could adapt to different needs of long-term storage and application, making them appear as a more stable solid state in a storage environment and a liquid state with good flowability in a use environment. Compared with a traditional positive temperature sensitive polymer material, the conversion temperature of these polymers was lower. These novel polymers were used as drug sustained-release carriers, and the injection temperature thereof was close to or lower than the human body temperature, which could simplify the injection operation and reduce local irritation and patient pain. A drug complex prepared from this class of novel polymers and suitable active therapeutic agents has been proven to have stable drug release behavior in an in vitro release experiment, and the drug release rate could also be flexibly controlled by adjusting the proportion of relevant structural units in the polymers.

A pharmaceutical composition prepared from these polymers maintained excellent drug release properties and degradability, while also taking into account the stability during storage and needle penetration during injection. In summary, this type of polymer and a drug delivery system thereof had the advantages of stable storage, convenient administration, stable drug release, and controllable degradation, and had significant value for application and research in the pharmaceutical field.

### BRIEF DESCRIPTION OF THE DRAWINGS

FIG. 1 shows one of the possible structures of an oligomer monomer synthesized from glycerol monostearate (GMS) and glycolide (GA) components with a molar ratio of 1:1 in Example 1 of the present invention.
FIG. 2 shows one of the possible structures of a polymer having the properties of acid-sensitive degradation and positive temperature sensitivity and synthesized from 3,9-di(ethylidene)-2,4,8,10-tetraoxaspiro[5,5]undecane (DETOSU), triethylene glycol (TEG), and glycerol monostearate diglycolide (GMS-diGL) components with a molar ratio (DETOSU: TEG: GMS-diGL) of 90:80:20 in Example 2 of the present invention.
FIG. 3 shows the mass spectrum of an oligomer synthesized from GMS and GA components with a molar ratio of 1:1 in Example 1 of the present invention.

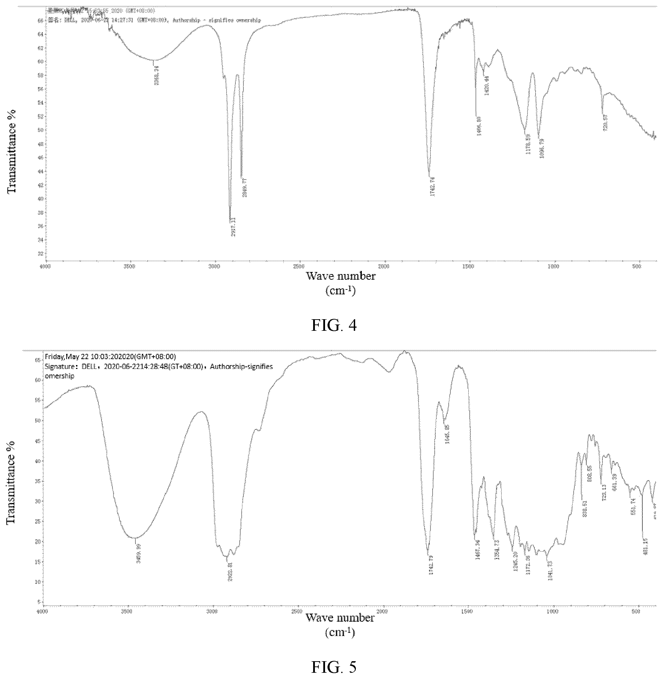

FIG. 4 shows the infrared spectrum of an oligomer monomer synthesized from GMS and GA components with a molar ratio of 1:1 in Example 1 of the present invention.
FIG. 5 shows the infrared spectrum of a polymer having the properties of acid-sensitive degradation and positive temperature sensitivity and synthesized from DETOSU, TEG and GMS-diGL components with a molar ratio (DETOSU: TEG: GMS-diGL) of 90:80:20 in Example 2 of the present invention.

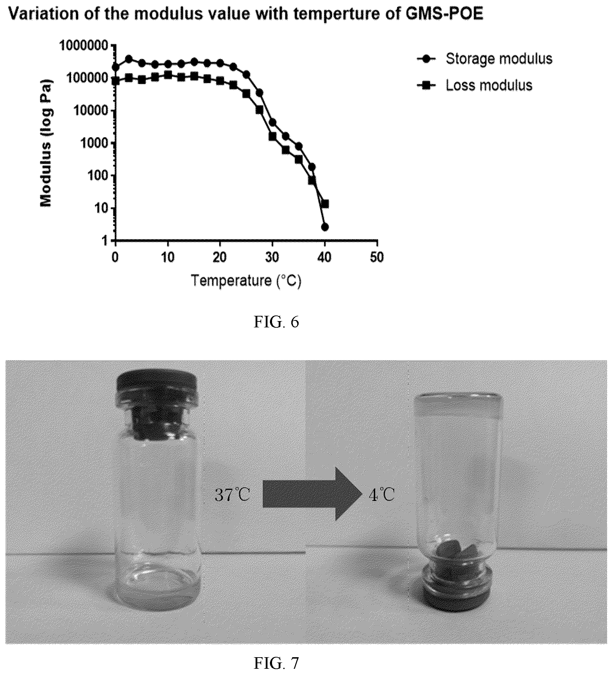

FIG. 6 shows the rheological property change of a polymer having the properties of acid-sensitive degradation and positive temperature sensitivity (GMS-POE) synthesized from DETOSU, TEG and GMS-diGL components with a molar ratio (DETOSU: TEG: GMS-diGL) of 90:80:20 at temperatures ranging from 0°C to 40°C in Example 2 of the present invention.
FIG. 7 shows the gel state of a polymer having the properties of acid-sensitive degradation and positive temperature sensitivity and synthesized from DETOSU, TEG and GMS-diGL components with a molar ratio (DETOSU: TEG: GMS-diGL) at 37°C (left) and 4°C (right) in Example 2 of the invention.
FIG. 8 shows the in vitro release curve of a drug complex containing 1.25% pramipexole prepared from the above-mentioned polymer having the properties of acid-sensitive degradation and positive temperature sensitivity at 37°C and pH 7.40 in Example 7 of the present invention.
FIG. 9 shows the in vitro release curve of a drug complex containing 3.3% granisetron prepared from the above-mentioned polymer having the properties of acid-sensitive degradation and positive temperature sensitivity at 37°C and pH 7.40 in Example 8 of the present invention.
FIG. 10 shows the composite viscosity variation curve of the series of novel polymers (GMS-POEs) provided by the present invention in the range of 0°C-40°C.

### DETAILED DESCRIPTION

The present invention will be further explained through examples. The present invention is not limited to the following examples, and various changes and equivalent substitutions can be made to the present invention within the scope of the claims of the present invention.

### Example 1 A preparation method of an oligomer monomer

The oligomer monomer of this example was prepared from glycerol monostearate (GMS) and glycolide (GA). A molar ratio of the two components was 1: 1.

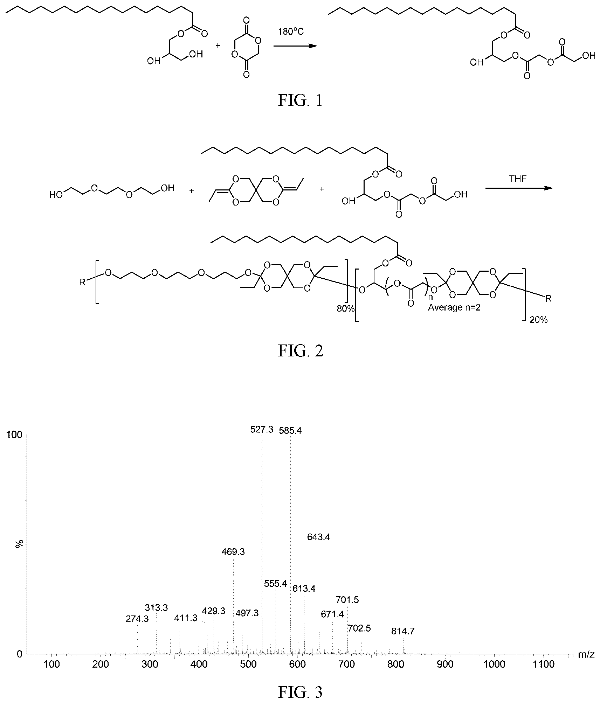

As shown in FIG. 1, glycerol monostearate (GMS) (17.928 g, 0.05 mol) and glycolide (GA) (5.8035 g, 0.05 mol) were weighed and placed in a pressure resistant reaction tube, heated and stirred in a closed manner at 180°C under inert gas protection for 24 h, and the resulting product was glycerol monostearate diglycolide (GMS-diGL).

### Example 2 A preparation method of a polymer having the properties of acid-sensitive degradation and positive temperature sensitivity

The polymer having the properties of acid-sensitive degradation and positive temperature sensitivity in this example was prepared from 3,9-di(ethylidene)-2,4,8,10-tetraoxaspiro[5,5]undecane (DETOSU), triethylene glycol (TEG), and glycerol monostearate diglycolide (GMS-diGL). A molar ratio of the three components (DETOSU: TEG: GMS-diGL) was 90:80:20.

As shown in FIG. 2, DETOSU (1.910 g, 0.009 mol) was dissolved in 15 ml anhydrous tetrahydrofuran (THF) and TEG (1.2014 g, 0.008 mol) in a 50 ml flask under a strict anhydrous condition, and GMS-diGL (0.9493 g, 0.002 mol) was dissolved in 5 ml anhydrous THF. The GMS-diGL solution was added to the DETOSU and TEG solutions to initiate polymerization reaction. Within a few minutes, the solution reached its boiling point. The solution was cooled to room temperature, then concentrated by rotary evaporation at 50°C, followed by rotary evaporation at 80°C.

### Example 3 A preparation method of a polymer having the properties of acid-sensitive degradation and positive temperature sensitivity

The polymer having the properties of acid-sensitive degradation and positive temperature sensitivity in this example was prepared from 3,9-di(ethylidene)-2,4,8,10-tetraoxaspiro[5,5]undecane (DETOSU), triethylene glycol (TEG), and glycerol monolaurate diglycolide (GML-diGL). A molar ratio of the three components (DETOSU: TEG: GML-diGL) was 95:80:20.

DETOSU (2.0164g, 0.0095mol) was dissolved in 15 ml anhydrous tetrahydrofuran (THF) and TEG (1.2014 g, 0.008 mol) in a 50 ml flask under a strict anhydrous condition, and GML-diGL (0.7807g, 0.002 mol) was dissolved in 5 ml anhydrous THF. The GML-diGL solution was added to the DETOSU and TEG solutions to initiate polymerization reaction. Within a few minutes, the solution reached its boiling point. The solution was cooled to room temperature, then concentrated by rotary evaporation at 50°C, followed by rotary evaporation at 80°C.

### Example 4 A preparation method of a polymer having the properties of acid-sensitive degradation and positive temperature sensitivity

The polymer having the properties of acid-sensitive degradation and positive temperature sensitivity in this example was prepared from 3,9-di(ethylidene)-2,4,8,10-tetraoxaspiro[5,5]undecane (DETOSU), triethylene glycol (TEG), and glyceryl monooleate diglycolide (GMO-diGL). A molar ratio of the three components (DETOSU: TEG: GMO-diGL) was 100:85:15.

DETOSU (2.1225g, 0.0100mol) was dissolved in 15 ml anhydrous ethyl acetate and TEG (1.2750g, 0.0085mol) in a 50 ml flask under a strict anhydrous condition, and GMO-diGL (0.7088g, 0.0015mol) was dissolved in 5 ml anhydrous ethyl acetate. The GMO-diGL solution was added to the DETOSU and TEG solutions to initiate polymerization reaction. Within a few minutes, the solution reached its boiling point. The solution was cooled to room temperature, then concentrated by rotary evaporation at 50°C, followed by rotary evaporation at 80°C.

### Example 5 A preparation method of a semi-solid drug complex

A semi-solid pharmaceutical composition using pramipexole (PPX) as an active agent was prepared by the following method:
1.25 wt% pramipexole was mixed with 98.75 wt% polymer under inert gas protection and heated at 40°C for 3 h, then cooled to room temperature to obtain a semi-solid with uniform texture.

### Example 6 A preparation method of a semi-solid drug complex

A semi-solid pharmaceutical composition using granisetron (GRA) as an active agent was prepared by the following method:
3.0 wt% granisetron was mixed with 97.0 wt% polymer under inert gas protection and heated at 40°C for 3 h, then cooled to room temperature to obtain a semi-solid with uniform texture.

### Example 7 The in vitro release properties of the pharmaceutical composition

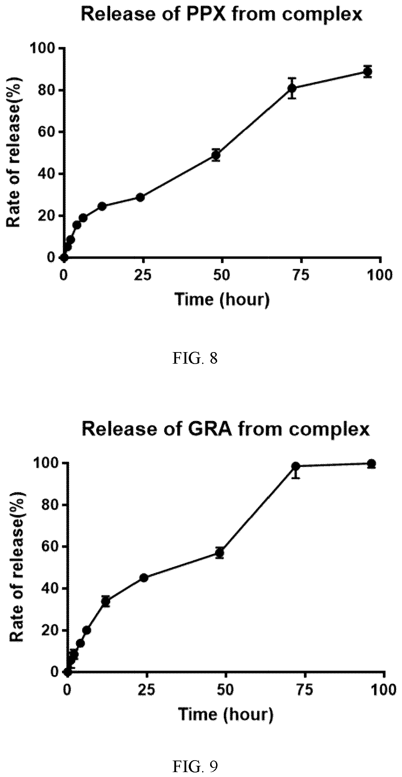

The pharmaceutical composition of Example 5 was weighed into a dialysis bag and placed in a test tube with a screw cap and containing 15 ml of 0.2 N PBS (pH 7.4) at 37°C. The above-mentioned test tube was sealed and stood at a constant temperature of 37°C. At different time points, the above-mentioned test tube was inverted several times, then 5 ml release solution was removed and an equal volume of release medium was added at 37°C. The content of pramipexole was determined in the release solution by HPLC, the release rate was calculated, and the release curve (FIG. 8) was plotted.

### Example 8 The in vitro release properties of the pharmaceutical composition

The pharmaceutical composition of Example 6 was weighed into a dialysis bag and placed in a test tube with a screw cap and containing 15 ml of 0.2 N PBS (pH 7.4) at 37°C. The above-mentioned test tube was sealed and stood at a constant temperature of 37°C. At different time points, the above-mentioned test tube was inverted several times, then 5 ml release solution was removed and an equal volume of release medium was added at 37°C. The content of granisetron was determined in the release solution by HPLC, the release rate was calculated, and the release curve (FIG. 9) was plotted.

## Claims

1. A polymer having properties of temperature sensitivity and a structure represented by formula (1), 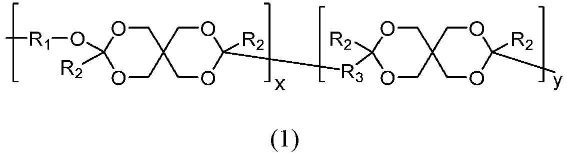
wherein: x and y are independently integers greater than 1;
R₁ is: 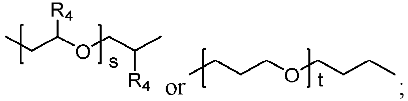
wherein:
s is an integer from 0 to 30;
t is an integer from 0 to 30;
R₄ is hydrogen or methyl;
R₂ is C₁₋₄ alkyl;
R₃ is: 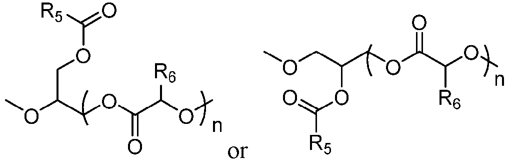
wherein:
R₅ is C₁₋₃₀ alkyl with or without a functional group, wherein the functional group is independently selected from a carbon-carbon double bond, a carbon-carbon triple bond, carbonyl, an aldehyde group, carboxyl, an ester bond, an amide bond, an ether bond, and amino; R₆ is hydrogen or C₁₋₄ alkyl; and
n is an integer from 1 to 20.

2. The polymer according to claim 1, wherein the polymer is polymerized from the following three monomers:
monomer A: 3,9-di(ethylidene)-2,4,8,10-tetraoxaspiro[5,5]undecane or a homologue thereof;
monomer B: a diol; and
monomer C: an oligomer monomer of formula (2) or (3): 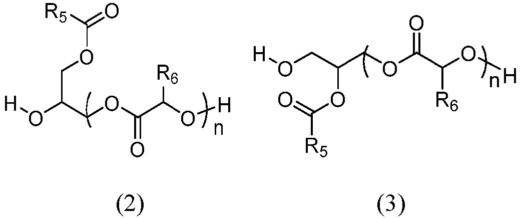
wherein:
R₅ is C₁₋₃₀ alkyl with or without a functional group, wherein the functional group is independently selected from a carbon-carbon double bond, a carbon-carbon triple bond, carbonyl, an aldehyde group, carboxyl, an ester bond, an amide bond, an ether bond, and amino;
R₆ is hydrogen or C₁₋₄ alkyl; and
n is an integer from 1 to 20.

3. The polymer according to claim 2, wherein
the monomer A: 3,9-di(ethylidene)-2,4,8,10-tetraoxaspiro[5,5]undecane; 3,9-di(propylidene)-2,4,8,10-tetraoxaspiro[5,5]undecane;
the monomer B: a diol with the following structural formula (4) or (5): 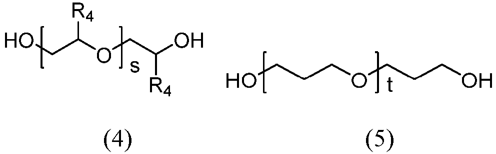
wherein:
s is an integer from 0 to 30;
t is an integer from 0 to 30;
R₄ is hydrogen or methyl; and
in the monomer C:
R₅ is C₁₋₃₀ alkyl with or without a functional group, wherein the functional group is independently selected from a carbon-carbon double bond, a carbon-carbon triple bond, carbonyl, an aldehyde group, carboxyl, and an ester bond;
R₆ is hydrogen or C₁₋₂ alkyl; and
n is an integer from 1 to 15.

4. The polymer according to claim 3, wherein
the monomer A: 3,9-di(ethylidene)-2,4,8,10-tetraoxaspiro[5,5]undecane;
in the monomer C:
R₅ is C₁₋₃₀ alkyl with or without a functional group, wherein the functional group is independently selected from a carbon-carbon double bond, a carbon-carbon triple bond, and
carbonyl;
R₆ is hydrogen or C₁₋₂ alkyl; and
n is an integer from 1 to 10.

5. The polymer according to claim 1, 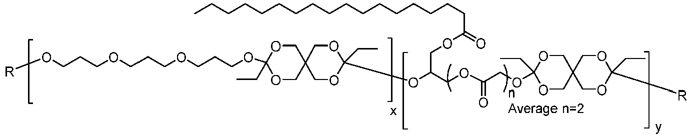 wherein: x and y are independently integers greater than 1.

6. A preparation method of the polymer according to claim 1, wherein polymerization is performed on monomer A: 3,9-di(ethylidene)-2,4,8,10-tetraoxaspiro[5,5]undecane or a homologue thereof;
monomer B: a diol with the following structural formula (4) or (5): 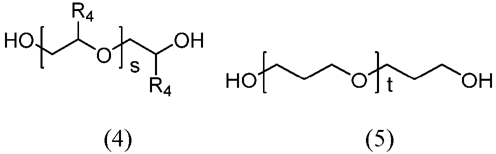
wherein:
s is an integer from 0 to 30;
t is an integer from 0 to 30;
R₄ is hydrogen or methyl; and
monomer C: an oligomer monomer of formula (2) or (3): 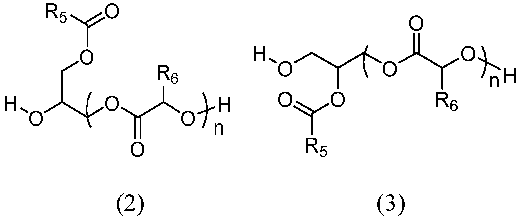
wherein:
R₅ is C₁₋₃₀ alkyl with or without a functional group, wherein the functional group is independently selected from a carbon-carbon double bond, a carbon-carbon triple bond, carbonyl, an aldehyde group, carboxyl, an ester bond, an amide bond, an ether bond, and amino; R₆ is hydrogen or C₁₋₄ alkyl; and
n is an integer from 1 to 20;
in the presence of an organic solvent at a reaction temperature of 0°C-300°C.

7. A composition comprising the polymer according to any one of claims 1-5.

8. The composition according to claim 7, further comprising one or more therapeutic active agents and pharmaceutically acceptable excipients, wherein the therapeutic active agent is a substance used for preventing, treating, and diagnosing human diseases, purposefully regulating human physiological functions, and specifying indications or major functions, usage, and dosage, and comprises traditional Chinese medicines, chemical drugs, and biological products; the therapeutic active agent is an antiparkinsonian drug, and selected from levodopa, carbidopa, nitecapone, bromocriptine, pramipexole, ropionitril, selegiline, benzoxetine, phenytoin, amantadine, and rotigotine;
the therapeutic active agent is an antiemetic agent, and selected from diphenhydramine, metoclopramide, scopolamine, benzoxetine, chlorpromazine, ondansetron, granisetron, metoclopramide, and domperidone;
the therapeutic active agent is a local anesthetic, and selected from procaine, tetracaine, lidocaine, and bupivacaine;
the therapeutic active agent is a nonsteroidal anti-inflammatory drug selected from aspirin, acetaminophen, indomethacin, ibuprofen, naproxen, and meloxicam;
the therapeutic active agent is a growth factor;
the therapeutic active agent is a gene-based drug; and
the therapeutic active agent is a protein drug or therapeutic peptide, and selected from insulin and glucagon-like peptide.

9. An oligomer monomer of structural formula (2) or (3): 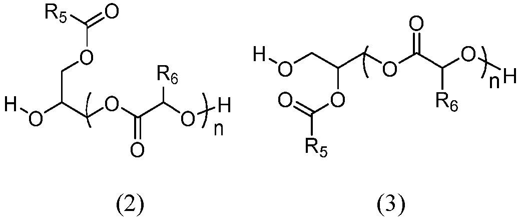 wherein:
R₅ is C₁₋₃₀ alkyl with or without a functional group, wherein the functional group is independently selected from a carbon-carbon double bond, a carbon-carbon triple bond, carbonyl, an aldehyde group, carboxyl, an ester bond, an amide bond, an ether bond, and amino;
R₆ is hydrogen or C₁₋₄ alkyl; and
n is an integer from 1 to 20.

10. A preparation method of the oligomer monomer according to claim 9, wherein the oligomer is prepared by polymerization of monomer D having the following structural formula (6) or (7) and monomer E (ethylene or propylene) under heating conditions; 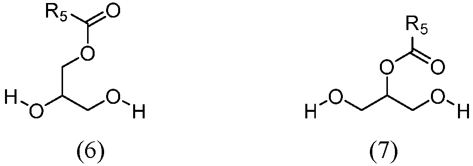 wherein:
R₅ is C₁₋₃₀ alkyl with or without a functional group, wherein the functional group is independently selected from a carbon-carbon double bond, a carbon-carbon triple bond, carbonyl, an aldehyde group, carboxyl, an ester bond, an amide bond, an ether bond, and amino;
and
a molar ratio of components D and E is 2:1 to 1:5.
